(19) Europäisches Patentamt
European Patent Office
Office européen des brevets

(11) EP 1 628 971 B1

(12) EUROPEAN PATENT SPECIFICATION

(45) Date of publication and mention of the grant of the patent:
**07.05.2008 Bulletin 2008/19**

(21) Application number: **04731152.7**

(22) Date of filing: **05.05.2004**

(51) Int Cl.:
***C07D 413/14*** (2006.01) ***A61K 31/4196*** (2006.01)
***A61P 7/02*** (2006.01)

(86) International application number:
**PCT/EP2004/004752**

(87) International publication number:
**WO 2004/101555 (25.11.2004 Gazette 2004/48)**

(54) **TRIAZOLE-DERIVATIVES AS FACTOR Xa INHIBITORS**

TRIAZOLDERIVATE ALS FAKTOR-Xa-INHIBITOREN

DERIVES DE TRIAZOLES EN TANT QU'INHIBITEURS DU FACTEUR Xa

(84) Designated Contracting States:
**AT BE BG CH CY CZ DE DK EE ES FI FR GB GR HU IE IT LI LU MC NL PL PT RO SE SI SK TR**

(30) Priority: **19.05.2003 EP 03011309**

(43) Date of publication of application:
**01.03.2006 Bulletin 2006/09**

(73) Proprietor: **Sanofi-Aventis Deutschland GmbH**
**65929 Frankfurt am Main (DE)**

(72) Inventors:
- **NAZARE, Marc**
  **65510 Idstein (DE)**
- **LAUX, Volker**
  **55128 Mainz (DE)**
- **BAUER, Armin**
  **65843 Sulzbach (Taunus) (DE)**
- **WAGNER, Michael**
  **64665 Alsbach (DE)**

(56) References cited:
**WO-A-01/32628** **WO-A-02/00651**
**WO-A-99/32454** **US-A- 5 998 424**
**US-A- 6 020 357** **US-A1- 2002 091 116**
**US-B1- 6 339 099**



Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

Printed by Jouve, 75001 PARIS (FR)

## Description

The present invention relates to compounds included in the general formulae I and II,

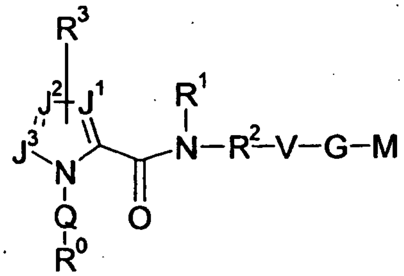

(I)

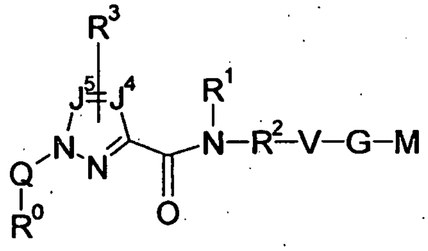

(II)

The compounds exhibit a strong anti-thrombotic effect and are suitable, for example, for the therapy and prophylaxis of cardio-vascular disorders like thromboembolic diseases or restenoses. They are reversible inhibitors of the blood clotting enzymes factor Xa (FXa) and/or factor VIIa (FVIIa), and can in general be applied in conditions in which an undesired activity of factor Xa and/or factor VIIa is present or for the cure or prevention of which an inhibition of factor Xa and/or factor VIIa is intended.

Normal haemeostasis is the result of a complex balance between the processes of clot initiation, formation and clot dissolution. The complex interactions between blood cells, specific plasma proteins and the vascular surface, maintain the fluidity of blood unless injury and blood loss occurs (EP-A-987274). Many significant disease states are related to abnormal haemeostasis. For example, local thrombus formation due to rupture of atheroslerotic plaque is a major cause of acute myocardial infarction and unstable angina. Treatment of an occlusive coronary thrombus by either thrombolytic therapy or percutaneous angioplasty may be accompanied by acute thrombolytic reclosure of the affected vessel.

There continues to be a need for safe and effective therapeutic anticoagulants to limit or prevent thrombus formation. It is most desirable to develop agents that inhibit coagulation without directly inhibiting thrombin but by inhibiting other steps in the coagulation cascade like factor Xa and/or factor VIIa activity. It is now believed that inhibitors of factor Xa carry a lower bleeding risk than thrombin inhibitors (A. E. P. Adang & J. B. M. Rewinkel, Drugs of the Future 2000, 25, 369-383).

Low molecular weight, factor Xa-specific blood clotting inhibitors that are effective but do not cause unwanted side effects have been described, for example, in WO-A-95/29189. WO 02/00651 also discloses Facfor Xa-inhibitors. However, besides being an effective factor Xa-specific blood clotting inhibitor, it is desirable that such inhibitors also have further advantageous properties, for instance stability in plasma and liver and selectivity versus other serine proteases whose inhibition is not intended, such as thrombin. There is an ongoing need for further low molecular weight factor Xa specific blood clotting inhibitors, which are effective and have the above advantages as well.

Specific inhibition of the factor VIIa/tissue factor catalytic complex using monoclonal antibodies (WO-A-92/06711) or a protein such as chloromethyl ketone inactivated factor VIIa (WO-A-96/12800, WO-A-97/47651) is an extremely effective means of controlling thrombus formation caused by acute arterial injury or the thrombotic complications related to bacterial septicemia. There is also experimental evidence suggesting that inhibition of factor VIIa/tissue factor activity inhibits restenosis following balloon angioplasty. Bleeding studies have been conducted in baboons and indicate that inhibition of the factor VIIa/tissue factor complex has the widest safety window with respect to therapeutic effectiveness and bleeding risk of any anticoagulant approach tested including thrombin, platelet and factor Xa inhibition. Certain inhibitors of factor VIIa have already been described. EP-A-987274, for example discloses compounds containing a tripeptide unit, which inhibit factor VIIa. However, the property profile of these compounds is still not ideal, and there is an ongoing need for further low molecular weight factor VIIa inhibitory blood clotting inhibitors

The present invention satisfies the above needs by providing novel compounds, which exhibit better factor Xa and/or factor VIIa inhibitory activity and are favorable agents with high bioavailability.

Thus, the present invention relates to a compound selected from the group consisting of:

1-[5-(5-Chloro-thiophen-2-yl)-isoxazol-3-ylmethyl]-5-pyrrol-1-yl-1H-[1,2,4]triazole-3-carboxylic acid (1-isopropyl-piperidin-4-yl)-amide,
1-[5-(5-Chloro-thiophen-2-yl)-isoxazol-3-ylmethyl]-5-pyridin-2-yl-1H-[1,2,4]triazole-3-carboxylic acid (1-isopropyl-piperidin-4-yl)-amide,
2-[5-(5-Chloro-thiophen-2-yl)-isoxazol-3-ylmethyl]-5-pyridin-2-yl-2H-[1,2,4]triazole-3-carboxylic acid (1-isopropyl-piperidin-4-yl)-amide,
1-[5-(5-Chloro-thiophen-2-yl)-isoxazol-3-ylmethyl]-5-phenyl-1H-[1,2,4]triazole-3-carboxylic acid (1-isopropyl-piperidin-4-yl)-amide or
2-[5-(5-Chloro-thiophen-2-yl)-isoxazol-3-ylmethyl]-2H-tetrazole-5-carboxylic acid (1-isopropyl-piperidin-4-yl)-amide.

Optically active carbon atoms present in said compounds can independently of each other have R configuration or S configuration. Said compounds can be present in the form of pure enantiomers or pure diastereomers or in the form of mixtures of enantiomers and/or diastereomers, for example in the form of racemates. The present invention relates to pure enantiomers and mixtures of enantiomers as well as to pure diastereomers and mixtures of diastereomers. The invention comprises mixtures of two or of more than two stereoisomers of said compounds, and it comprises all ratios of the stereoisomers in the mixtures. In case said compounds can be present as E isomers or Z isomers (or cis isomers or trans isomers) the invention relates both to pure E isomers and pure Z isomers and to E/Z mixtures in all ratios. The invention also comprises all tautomeric forms of said compounds.

Diastereomers, including E/Z isomers, can be separated into the individual isomers, for example, by chromatography. Racemates can be separated into the two enantiomers by customary methods, for example by chromatography on chiral phases or by resolution, for example by crystallization of diastereomeric salts obtained with optically active acids or bases. Stereochemically uniform compounds can also be obtained by employing stereochemically uniform starting materials or by using stereoselective reactions.

Physiologically tolerable salts of said compounds are nontoxic salts that are physiologically acceptable, in particular pharmaceutically utilizable salts. Such salts of compounds containing acidic groups, for example a carboxyl group COOH, are for example alkali metal salts or alkaline earth metal salts such as sodium salts, potassium salts, magnesium salts and calcium salts, and also salts with physiologically tolerable quaternary ammonium ions such as tetramethylammonium or tetraethylammonium, and acid addition salts with ammonia and physiologically tolerable organic amines, such as methylamine, dimethylamine, trimethylamine, ethylamine, triethylamine, ethanolamine or tris-(2-hydroxyethyl)amine. Basic groups contained in said compounds, for example amino groups or guanidino groups, form acid addition salts, for example with inorganic acids such as hydrochloric acid, hydrobromic acid, sulfuric acid, nitric acid or phosphoric acid, or with organic carboxylic acids and sulfonic acids such as formic acid, acetic acid, oxalic acid, citric acid, lactic acid, malic acid, succinic acid, malonic acid, benzoic acid, maleic acid, fumaric acid, tartaric acid, methanesulfonic acid or p-toluenesulfonic acid. Compounds, which simultaneously contain a basic group and an acidic group, for example a guanidino group and a carboxyl group, can also be present as zwitterions (betaines), which are likewise included in the present invention.

Salts of said compounds can be obtained by customary methods known to those skilled in the art, for example by combining a compound with an inorganic or organic acid or base in a solvent or dispersant, or from other salts by cation exchange or anion exchange.

The present invention furthermore includes all solvates of said compounds, for example hydrates or adducts with alcohols.

The compounds of the present invention are serine protease inhibitors, which inhibit the activity of the blood coagulation enzyme factors Xa and/or factor VIIa. In particular, they are highly active inhibitors of factor Xa. They are specific serine protease inhibitors inasmuch as they do not substantially inhibit the activity of other proteases whose inhibition is not desired. The activity of the compounds can be determined, for example, in the assays described below or in other assays known to those skilled in the art. With respect to factor Xa inhibition, a preferred embodiment of the invention comprises compounds, which have a Ki ≤ 1 mM for factor Xa inhibition as determined in the assay described below, with or without concomitant factor VIIa inhibition, and which preferably do not substantially inhibit the activity of other proteases involved in coagulation and fibrinolysis whose inhibition is not desired (using the same concentration of the inhibitor). The compounds of the invention inhibit factor Xa catalytic activity either directly, within the prothrombinase complex or as a soluble subunit, or indirectly, by inhibiting the assembly of factor Xa into the prothrombinase complex.

The present invention also relates to said compounds and/or their physiologically tolerable salts for use as pharmaceuticals (or medicaments), or to the use of said compounds and/or their physiologically tolerable salts for the production of pharmaceuticals for inhibition of factor Xa and/or factor VIIa or for influencing blood coagulation, inflammatory response or fibrinolysis or for the therapy or prophylaxis of the diseases mentioned above or below, for example for the production of pharmaceuticals for the therapy and prophylaxis of cardiovascular disorders, thromboembolic diseases or restenoses. The invention also relates to said compounds and/or their physiologically tolerable salts for the inhibition of factor Xa and/or factor VIIa or for influencing blood coagulation or fibrinolysis or for the therapy or prophylaxis of the diseases mentioned above or below, for example for use in the therapy and prophylaxis of cardiovascular disorders, thromboembolic diseases or restenoses, and to methods of treatment aiming at such purposes including methods for said therapies and prophylaxis. The present invention also relates to pharmaceutical preparations (or pharmaceutical compositions) which contain an effective amount of at least one of said compounds and/or its physiologically tolerable salts in addition to a customary pharmaceutically acceptable carrier, i. e. one or more pharmaceutically acceptable carrier substances or excipients and/or auxiliary substances or additives.

The compounds can be used in the treatment of disease states such as abnormal thrombus formation, acute myocardial infarction, unstable angina, thromboembolism, acute vessel closure associated with thrombolytic therapy or

percutaneous transluminal coronary angioplasty, transient ischemic attacks, stroke, pathologic thrombus formation occurring in the veins of the lower extremities following abdominal, knee and hip surgery, a risk of pulmonary thromboembolism, or disseminated systemic intravascular coagulatopathy occurring in vascular systems during septic shock, certain viral infections or cancer.

Said compounds and their physiologically tolerable salts can be administered to animals, preferably to mammals, and in particular to humans as pharmaceuticals for therapy or prophylaxis. They can be administered on their own, or in mixtures with one another or in the form of pharmaceutical preparations, which permit enteral or parenteral administration.

The pharmaceuticals can be administered orally, for example in the form of pills, tablets, lacquered tablets, coated tablets, granules, hard and soft gelatin capsules, solutions, syrups, emulsions, suspensions or aerosol mixtures. Administration, however, can also be carried out rectally, for example in the form of suppositories, or parenterally, for example intravenously, intramuscularly or subcutaneously, in the form of injection solutions or infusion solutions, microcapsules, implants or rods, or percutaneously or topically, for example in the form of ointments, solutions or tinctures, or in other ways, for example in the form of aerosols or nasal sprays.

The pharmaceutical preparations according to the invention are prepared in a manner known per se and familiar to one skilled in the art, pharmaceutically acceptable inert inorganic and/or organic carriers being used in addition to said compound(s) and/or its (their) physiologically tolerable salts. For the production of pills, tablets, coated tablets and hard gelatin capsules it is possible to use, for example, lactose, cornstarch or derivatives thereof, talc, stearic acid or its salts, etc. Carriers for soft gelatin capsules and suppositories are, for example, fats, waxes, semisolid and liquid polyols, natural or hardened oils, etc. Suitable carriers for the production of solutions, for example injection solutions, or of emulsions or syrups are, for example, water, saline, alcohols, glycerol, polyols, sucrose, invert sugar, glucose, vegetable oils, etc. Suitable carriers for microcapsules, implants or rods are, for example, copolymers of glycolic acid and lactic acid. The pharmaceutical preparations normally contain about 0.5 % to 90 % by weight of said compounds and/or their physiologically tolerable salts. The amount of the active ingredient and/or its physiologically tolerable salts in the pharmaceutical preparations normally is from about 0.5 mg to about 1000 mg, preferably from about 1 mg to about 500 mg.

In addition to the active ingredients of said compounds and/or their physiologically acceptable salts and to carrier substances, the pharmaceutical preparations can contain additives such as, for example, fillers, disintegrants, binders, lubricants, wetting agents, stabilizers, emulsifiers, preservatives, sweeteners, colorants, flavorings, aromatizers, thickeners, diluents, buffer substances, solvents, solubilizers, agents for achieving a depot effect, salts for altering the osmotic pressure, coating agents or antioxidants. They can also contain two or more of said compounds and/or their physiologically tolerable salts. In case a pharmaceutical preparation contains two or more of said compounds the selection of the individual compounds can aim at a specific overall pharmacological profile of the pharmaceutical preparation. For example, a highly potent compound with a shorter duration of action may be combined with a long-acting compound of lower potency. The flexibility permitted with respect to the choice of substituents in said compounds allows a great deal of control over the biological and physico-chemical properties of the compounds and thus allows the selection of such desired compounds. Furthermore, in addition to at least one of said compound and/or its physiologically tolerable salts, the pharmaceutical preparations can also contain one or more other therapeutically or prophylactically active ingredients.

As inhibitors of factor Xa and/or factor VIIa said compounds and their physiologically tolerable salts are generally suitable for the therapy and prophylaxis of conditions in which the activity of factor Xa and/or factor VIIa plays a role or has an undesired extent, or which can favorably be influenced by inhibiting factor Xa and/or factor VIIa or decreasing their activities, or for the prevention, alleviation or cure of which an inhibition of factor Xa and/or factor VIIa or a decrease in their activity is desired by the physician. As inhibition of factor Xa and/or factor VIIa influences blood coagulation and fibrinolysis, said compounds and their physiologically tolerable salts are generally suitable for reducing blood clotting, or for the therapy and prophylaxis of conditions in which the activity of the blood coagulation system plays a role or has an undesired extent, or which can favorably be influenced by reducing blood clotting, or for the prevention, alleviation or cure of which a decreased activity of the blood coagulation system is desired by the physician. A specific subject of the present invention thus are the reduction or inhibition of unwanted blood clotting, in particular in an individual, by administering an effective amount of said compound or a physiologically tolerable salt thereof, as well as pharmaceutical preparations therefor.

Conditions in which said compounds can be favorably used include, for example, cardiovascular disorders, thromboembolic diseases or complications associated, for example, with infection or surgery. The compounds of the present invention can also be used to reduce an inflammatory response. Examples of specific disorders for the treatment or prophylaxis of which said compounds can be used are coronary heart disease, myocardial infarction, angina pectoris, vascular restenosis, for example restenosis following angioplasty like PTCA, adult respiratory distress syndrome, multiorgan failure, stroke and disseminated intravascular clotting disorder. Examples of related complications associated with surgery are thromboses like deep vein and proximal vein thrombosis, which can occur following surgery. In view of their pharmacological activity the compounds of the invention can replace or supplement other anticoagulant agents such as heparin. The use of a compound of the invention can result, for example, in a cost saving as compared to other

anticoagulants. When using said compounds the dose can vary within wide limits and, as is customary and is known to the physician, is to be suited to the individual conditions in each individual case. It depends, for example, on the specific compound employed, on the nature and severity of the disease to be treated, on the mode and the schedule of administration, or on whether an acute or chronic condition is treated or whether prophylaxis is carried out. An appropriate dosage can be established using clinical approaches well known in the medical art. In general, the daily dose for achieving the desired results in an adult weighing about 75 kg is from 0.01 mg/kg to 100 mg/kg, preferably from 0.1 mg/kg to 50 mg/kg, in particular from 0.1 mg/kg to 10 mg/kg, (in each case in mg per kg of body weight). The daily dose can be divided, in particular in the case of the administration of relatively large amounts, into several, for example 2, 3 or 4, part administrations. As usual, depending on individual behavior it may be necessary to deviate upwards or downwards from the daily dose indicated.

Said compounds can also advantageously be used as an anticoagulant outside an individual. For example, an effective amount of a compound of the invention can be contacted with a freshly drawn blood sample to prevent coagulation of the blood sample. Further, said compounds and its salts can be used for diagnostic purposes, for example in in vitro diagnoses, and as an auxiliary in biochemical investigations. For example, said compounds can be used in an assay to identify the presence of factor Xa and/or factor VIIa or to isolate factor Xa and/or factor VIIa in a substantially purified form. A compound of the invention can be labeled with, for example, a radioisotope, and the labeled compound bound to factor Xa and/or factor VIIa is then detected using a routine method useful for detecting the particular label. Thus, said compounds or a salt thereof can be used as a probe to detect the location or amount of factor Xa and/or factor VIIa activity in vivo, in vitro or ex vivo.

The general synthetic sequences for preparing the compounds useful in the present invention our outlined in the examples given below. Both an explanation of, and the actual procedure for, the various aspects of the present invention are described where appropriate.

Examples

When in the final step of the synthesis of a compound an acid such as trifluoroacetic acid or acetic acid was used, for example when trifluoroacetic acid was employed to remove a tBu group or when a compound was purified by chromatography using an eluent which contained such an acid, in some cases, depending on the work-up procedure, for example the details of a freeze-drying process, the compound was obtained partially or completely in the form of a salt of the acid used, for example in the form of the acetic acid salt or trifluoroacetic acid salt or hydrochloric acid salt.

Abbreviations used:

| | |
|---|---|
| tert-Butyl | tBu |
| 2,2'-bis(diphenylphoshino-1,1'-binaphthyl | Binap |
| Bis-(oxo-3-oxazolidinyl)-phosphoryl chloride | BOP-Cl |
| dibenzylidenacetone | dba |
| Dichloromethane | DCM |
| Dicyclohexyl-carbodiimide | DCC |
| Diethylphosphoryl cyanide | DEPC |
| Diisopropylethyl amine | DIPEA |
| 4-Dimethyaminopyridine | DMAP |
| N,N-Dimethylformamide | DMF |
| Dimethylsulfoxide | DMSO |
| 1,1'-Bis(diphenylphosphino)ferrocene | DPPF |
| O-(7-Azabenzotriazol-1-yl)-N,N,N',N'-tetramethyluronium-hexafluorophosphate | HATU |
| N-Bromosuccinimide | NBS |
| N-Chlorosuccinimide | NCS |
| N-Iodosuccinimide | NIS |
| N-Ethylmorpholine | NEM |
| Methanol | MeOH |
| Room temperature 20 °C to 25 °C | RT |

(continued)

| | |
|---|---|
| Saturated | sat. |
| Tetrahydrofuran | THF |
| Trifluoroacetic acid | TFA |
| O-((Ethoxycarbonyl)cyanomethyleneamino)-N,N,N',N'-tetramethyluronium tetrafluoroborate | TOTU |

Example 1: 1-[5-(5-Chloro-thiophen-2-yl)-isoxazol-3-ylmethyl]-5-pyrrol-1-yl-1H-[1,2,4]triazole-3-carboxylic acid (1-isopropyl-piperidin-4-yl)-amide

(i) (1-Isopropyl-piperidin-4-yl)-carbamic acid tert-butyl ester

To a solution of 5.0 g Piperidin-4-yl-carbamic acid tert-butyl ester in 15 ml methanol 7.34 ml acetone, 3.14 g $Na(CN)BH_3$ and 0.3 ml acetic acid were added. After stirring for 16 h at RT the solvent was removed under reduced pressure and the residue was partitioned between 30 ml of water and 30 ml of ethyl acetate. The organic layer was washed with saturated $Na_2CO_3$ solution, water and then dried over $Na_2SO_4$. Following filtration, the solvent was removed under reduced pressure to yields a white solid. Yield: 4.8g MS ($ES^+$): m/e= 243.

(ii) 1-Isopropyl-piperidin-4-ylamine

To 4.8 g (1-Isopropyl-piperidin-4-yl)-carbamic acid tert-butyl ester in 15 ml methanol, 20 ml methanolic hydrochloric acid (8M) were added and the mixture was stirred for 16 h. Removal of the solvent under reduced pressure yielded a white solid, which was coevaporated twice with 20 ml toluene. The product was obtained as its hydrochloride. Yield: 5.42 g MS ($ES^+$): m/e= 143.

(iii) 5-Pyrrol-1-yl-1H-[1,2,4]triazole-3-carboxylic acid (1-isopropyl-piperidin-4-yl)-amide

To 100 mg 5-Pyrrol-1-yl-1H-[1,2,4]triazole-3-carboxylic acid in 1 ml DMF and 0.3 ml $NEt_3$, 121 mg 1-Isopropyl-piperidin-4-ylamine dihydrochloride and 142 mg·BOP-Cl were added at RT and the mixture was stirred for 16 h. After addition of 5 ml of water the mixture was filtered through a chem elut® cartridge by elution with ethyl acetate and then concentrated under reduced pressure. The obtained crude product was used without further purification in the next reaction step. Yield: 170 mg.

(iv) 1-[5-(5-Chloro-thiophen-2-yl)-isoxazol-3-ylmethyl]-5-pyrrol-1-yl-1H-[1,2,4]triazole-3-carboxylic acid (1-isopropyl-piperidin-4-yl)-amide

To a solution of 170 5-Pyrrol-1-yl-1H-[1,2,4]triazole-3-carboxylic acid (1-isopropyl-piperidin-4-yl)-amide in 1 ml DMF, 156 mg 3-Bromomethyl-5-(5-chloro-thiophen-2-yl)-isoxazole [prepared by adopting a procedure described by Ewing, William R.; Becker, Michael R.; Choi-Sledeski, Yong Mi; Pauls, Heinz W.; He, Wei; Condon, Stephen M.; Davis, Roderick S.; Hanney, Barbara A.; Spada, Alfred P.; Burns, Christopher J.; Jiang, John Z.; Li, Aiwen; Myers, Michael R.; Lau, Wan F.; Poli, Gregory B; PCT Int. Appl. (2001) 460 pp. WO 0107436 A2] and 183 mg $Cs_2CO_3$ were added and the mixture was stirred at room temperature for 1.5 h. After the addition of 2 ml sat. $NaHCO_3$ the mixture was filtered through a chem elut® cartridge by elution with ethyl acetate and then concentrated under reduced pressure. The residue was purified by preparative HPLC (C18 reverse phase column, elution with a $H_2O$/MeCN gradient with 0.1% TFA). The fractions containing the product were evaporated and lyophilized to yield a white solid. The product was obtained as its trifluoroacetate salt. Yield: 42 mg MS ($ES^+$): m/e =500, chloro pattern.

Example 2: 1-[5-(5-Chloro-thiophen-2-yl)-isoxazol-3-ylmethyl]-5-pyridin-2-yl-1H-[1,2,4]triazole-3-carboxylic acid (1-isopropyl-piperidin-4-yl)-amide

The title compound was prepared analogously to example 1 with the difference that 5-Pyridin-2-yl-1H-[1,2,4]triazole-3-carboxylic acid was used instead of 5-Pyrrol-1-yl-1H-[1,2,4]triazole-3-carboxylic acid. MS ($ES^+$): m/e = 512, chloro pattern.

Example 3: 2-[5-(5-Chloro-thiophen-2-yl)-isoxazol-3-ylmethyl]-5-pyridin-2-yl-2H-[1,2,4]triazole-3-carboxylic acid (1-isopropyl-piperidin-4-yl)-amide

This compound was isolated as a by-product in example 2.
MS ($ES^+$): m/e = 512, chloro pattern.

Example 4: 1-[5-(5-Chloro-thiophen-2-yl)-isoxazol-3-ylmethyl]-5-phenyl-1H-[1,2,4]triazole-3-carboxylic acid (1-isopropyl-piperidin-4-yl)-amide

The title compound was prepared analogously to example 1 with the difference that 5-Phenyl-1H-[1,2,4]triazole-3-carboxylic acid was used instead of 5-Pyrrol-1-yl-1H-[1,2,4]triazole-3-carboxylic acid. MS ($ES^+$): m/e = 511, chloro pattern.

Example 5: 2-[5-(5-Chloro-thiophen-2-yl)-isoxazol-3-ylmethyl]-2N-tetrazole-5-carboxylic acid (1-isopropyl-piperidin-4-yl)-amide

(i) 2-[5-(5-Chloro-thiophen-2-yl)-isoxazol-3-ylmethyl]-2H-tetrazole-5-carboxylic acid

To a solution of 100 mg 2H-Tetrazole-5-carboxylic acid ethyl ester sodium salt in 1 ml DMF, 59 mg 3-Bromomethyl-5-(5-chloro-thiophen-2-yl)-isoxazole [prepared by adopting a procedure described by Ewing, William R.; Becker, Michael R.; Choi-Sledeski, Yong Mi; Pauls, Heinz W.; He, Wei; Condon, Stephen M.; Davis, Roderick S.; Hanney, Barbara A.; Spada, Alfred P.; Burns, Christopher J.; Jiang, John Z.; Li, Aiwen; Myers, Michael R.; Lau, Wan F.; Poli, Gregory B; PCT Int. Appl. (2001) 460 pp. WO 0107436 A2] were added and the mixture was stirred at room temperature for 16 h. Then the reaction mixture was concentrated under reduced pressure. The residue was taken-up in 1 ml THF and after addtion of 1 ml of a 1 M NaOH solution stirred for 16 h at RT. The mixture was acidified with half concentrated hydrochloric acid to pH 3 and the precipitate collected by filtration. The product was obtained as a white solid, which was dried under reduced pressure. Yield: 49 mg.

(ii) 2-[5-(5-Chloro-thiophen-2-yl)-isoxazol-3-ylmethyl]-2H-tetrazole-5-carboxylic acid (1-isopropyl-piperidin-4-yl)-amide

To a solution of 49 mg 2-[5-(5-Chloro-thiophen-2-yl)-isoxazol-3-ylmethyl]-2H-tetrazole-5-carboxylic acid, 0.1 ml N-NEM in 1 ml DCM, 53 mg TOTU were added and the mixture was stirred for 5 min at RT. Then 34 mg 1-Isopropyl-piperidin-4-ylamine hydrochloride were added and the reaction was further stirred for 2 h. The reaction mixture was concentrated under reduced pressure and the residue was directly purified by preparative HPLC (C18 reverse phase column, elution with a $H_2O$/MeCN gradient with 0.1% TFA). The fractions containing the product were evaporated and lyophilized to yield a white solid. The product was obtained as its trifluoroacetate salt. Yield: 18 mg MS ($ES^+$): m/e = 436, chloro pattern.

Pharmacological testing

The ability of the compounds of the formula I or II to inhibit factor Xa or factor VIIa or other enzymes like thrombin, plasmin, or trypsin can be assessed by determining the concentration of the compound of the formula I or II that inhibits enzyme activity by 50 %, i. e. the IC50 value, which was related to the inhibition constant Ki. Purified enzymes were used in chromogenic assays. The concentration of inhibitor that causes a 50 % decrease in the rate of substrate hydrolysis was determined by linear regression after plotting the relative rates of hydrolysis (compared to the uninhibited control) versus the log of the concentration of the compound of formulae I, II, Ia, Ib, Ic, Id, IIa, IIb or IId. For calculating the inhibition constant Ki, the IC50 value was corrected for competition with substrate using the formula

$$Ki = IC50 / \{1 + (\text{substrate concentration} / Km)\}$$

wherein Km is the Michaelis-Menten constant (Chen and Prusoff, Biochem. Pharmacol. 22 (1973) 3099-3108; I. H. Segal, Enzyme Kinetics, 1975, John Wiley & Sons, New York, 100-125; which were incorporated herein by reference).

a) Factor Xa Assay

In the assay for determining the inhibition of factor Xa activity TBS-PEG buffer (50 mM Tris-HCl, pH 7.8, 200

mM NaCl, 0.05 % (w/v) PEG-8000, 0.02 % (w/v) $NaN_3$) was used. The IC50 was determined by combining in appropriate wells of a Costar half-area microtiter plate 25 μl human factor Xa (Enzyme Research Laboratories, Inc.; South Bend, Indiana) in TBS-PEG; 40 μl 10 % (v/v) DMSO in TBS-PEG (uninhibited control) or various concentrations of the compound to be tested diluted in 10 % (v/v) DMSO in TBS-PEG; and substrate S-2765 (N(α)-benzyloxycarbonyl-D-Arg-Gly-L-Arg-p-nitroanilide; Kabi Pharmacia, Inc.; Franklin, Ohio) in TBS-PEG.

The assay was performed by pre-incubating the compound of formula I or II plus enzyme for 10 min. Then the assay was initiated by adding substrate to obtain a final volume of 100 μl. The initial velocity of chromogenic substrate hydrolysis was measured by the change in absorbance at 405 nm using a Bio-tek Instruments kinetic plate reader (Ceres UV900HDi) at 25 °C during the linear portion of the time course (usually 1.5 min after addition of substrate).

The enzyme concentration was 0.5 nM and substrate concentration was 140 μM.

b) Factor VIIa Assay

The inhibitory activity towards factor VIIa/tissue factor activity was determined using a chromogenic assay essentially as described previously (J. A. Ostrem et al., Biochemistry 37 (1998) 1053-1059 which was incorporated herein by reference). Kinetic assays were conducted at 25 °C in half-area microtiter plates (Costar Corp., Cambridge, Massachusetts) using a kinetic plate reader (Molecular Devices Spectramax 250). A typical assay consisted of 25 μl human factor VIIa and TF (5 nM and 10 nM, respective final concentration) combined with 40 μl of inhibitor dilutions in 10% DMSO/TBS-PEG buffer (50 mM Tris, 15 mM NaCl, 5 mM $CaCl_2$, 0.05 % PEG 8000, pH 8.15). Following a 15 minute preincubation period, the assay was initiated by the addition of 35 μl of the chromogenic substrate S-2288 (D-Ile-Pro-Arg-p-nitroanilide, Pharmacia Hepar Inc., 500 μM final concentration). The results (inhibition constants Ki (FXa) for inhibition of factor Xa) are shown in Table 1.

Table1:

| Example | Ki(FXa) [μM] | Example | Ki(FXa) [μM] |
|---|---|---|---|
| 1 | 0.054 | 3 | 0.100 |
| 2 | 0.080 | 5 | 0.330 |

## Claims

1. A compound selected from the group consisting of:

   1-[5-(5-Chloro-thiophen-2-yl)-isoxazol-3-ylmethyl]-5-pyrrol-1-yl-1H-[1,2,4]triazole-3-carboxylic acid (1-isopropyl-piperidin-4-yl)-amide,
   1-[5-(5-Chloro-thiophen-2-yl)-isoxazol-3-ylmethyl]-5-pyridin-2-yl-1H-[1,2,4]triazole-3-carboxylic acid (1-isopropyl-piperidin-4-yl)-amide,
   2-[5-(5-Chloro-thiophen-2-yl)-isoxazol-3-ylmethyl]-5-pyridin-2-yl-2H-[1,2,4]triazole-3-carboxylic acid (1-isopropyl-piperidin-4-yl)-amide,
   1-[5-(5-Chloro-thiophen-2-yl)-isoxazol-3-ylmethyl]-5-phenyl-1H-[1,2,4]triazole-3-carboxylic acid (1-isopropyl-piperidin-4-yl)-amide or
   2-[5-(5-Chloro-thiophen-2-yl)-isoxazol-3-ylmethyl]-2H-tetrazole-5-carboxylic acid (1-isopropyl-piperidin-4-yl)-amide.

2. A pharmaceutical preparation, comprising a compound as claimed in claim 1 in all its stereoisomeric forms and mixtures thereof in any ratio and/or its physiologically tolerable salts and a pharmaceutically acceptable carrier.

3. The use of a compound as claimed in claim 1 in all its stereoisomeric forms and mixtures thereof in any ratio and/or their physiologically tolerable salts for the production of pharmaceuticals for influencing blood coagulation or fibrinolysis.

4. The use as claimed in claim 3 for abnormal thrombus formation, acute myocardial infarction, cardiovascular disorders, unstable angina, thromboembolism, acute vessel closure associated with thrombolytic therapy or percutaneous transluminal coronary angioplasty (PTCA), transient ischemic attacks, stroke, intermittent claudication, bypass grafting of the coronary or peripheral arteries, vessel luminal narrowing, restenosis post coronary or venous angioplasty, maintenance of vascular access patency in long-term hemodialysis patients, pathologic thrombus formation occurring

in the veins of the lower extremities following abdominal, knee or hip surgery, pathologic thrombus formation occurring in the veins of the lower extremities following abdominal, knee and hip surgery, a risk of pulmonary thromboembolism, or disseminated systemic intravascular coagulatopathy occurring in vascular systems during septic shock, viral infections or cancer, or reducing an inflammatory response, fibrinolysis, or treatment of coronary heart disease, myocardial infarction, angina pectoris, vascular restenosis, for example restenosis following angioplasty like PTCA, adult respiratory distress syndrome, multi-organ failure and disseminated intravascular clotting disorder, deep vein or proximal vein thrombosis, which can occur following surgery.

## Patentansprüche

**1.** Verbindung aus der Gruppe bestehend aus:

1-[5-(5-Chlorthiophen-2-yl)isoxazol-3-ylmethyl]-5-pyrrol-1-yl-1H-[1,2,4]triazol-3-carbonsäure-(1-isopropylpiperidin-4-yl)amid,
1-[5-(5- Chlorthiophen- 2- yl) isoxazol- 3- ylmethyl]- 5- pyridin- 2- yl- 1H-[1,2,4] triazol- 3- carbonsäure-(1- isopropylpiperidin-4-yl)amid,
2-[5-(5- Chlorthiophen- 2- yl) isoxazol- 3- ylmethyl]- 5- pyridin- 2- yl- 2H-[1,2,4] triazol- 3- carbonsäure-(1- isopropylpiperidin-4-yl)amid,
1-[5-(5-Chlorthiophen-2-yl)isoxazol-3-ylmethyl]-5-phenyl-1H-[1,2,4]triazol-3-carbonsäure-(1-isopropylpiperidin-4-yl)amid oder
2-[5-(5-Chlorthiophen-2-yl)isoxazol-3-ylmethyl]-2H-tetrazol-5-carbonsäure-(1-isopropylpiperidin-4-yl)amid.

**2.** Pharmazeutische Zubereitung, enthaltend eine Verbindung nach Anspruch 1 in allen ihren stereoisomeren Formen und Gemischen davon in beliebigem Verhältnis und/oder ihre physiologisch verträglichen Salze und einen pharmazeutisch unbedenklichen Träger.

**3.** Verwendung einer Verbindung nach Anspruch 1 in allen ihren stereoisomeren Formen und Gemischen davon in beliebigem Verhältnis und/oder ihrer physiologisch verträglichen Salze zur Herstellung von Pharmazeutika zur Beeinflussung von Blutgerinnung oder Fibrinolyse.

**4.** Verwendung nach Anspruch 3 für abnormale Thrombusbildung, akuten Herzinfarkt, kardiovaskuläre Störungen, instabile Angina, Thromboembolismus, akuten Gefäßverschluß assoziiert mit thrombolytischer Therapie oder perkutaner transluminaler Koronarangioplastie (PTCA), transitorische ischämische Attacken, Schlaganfall, Claudicatio intermittens, Bypassoperation der koronaren oder peripheren Arterien, luminale Gefäßverengung, Restenose nach Koronar- oder Venenangioplastie, Aufrechterhaltung der vaskulären Zugangsoffenheit bei Langzeithämodialysepatienten, pathologische Thrombusbildung, die in den Venen der unteren Extremitäten nach Bauch-, Knie- und Hüftoperationen auftritt, Lungenthromboembolismus-Risiko oder disseminierte systemische intravaskuläre Koagulopathie, die in Gefäßsystemen bei septischem Schock, Virusinfektionen oder Krebs auftritt, oder Verringerung einer Entzündungsreaktion, Fibrinolyse oder Behandlung von koronarer Herzkrankheit, Herzinfarkt, Angina pectoris, Gefäßrestenose, beispielsweise Restenose nach Angioplastie wie PTCA, Schocklunge, multiplem Organversagen und disseminierter intravaskulärer Gerinnung, tiefer Venenthrombose oder proximaler Venenthrombose, die nach Operationen auftreten kann.

## Revendications

**1.** Composé choisi dans le groupe constitué de :

le (1-isopropylpipéridin-4-yl)amide d'acide 1-[5-(5-chlorothiophén-2-yl)isoxazol-3-ylméthyl]-5-pyrrol-1-yl-1H-[1,2,4]triazole-3-carboxylique,
le (1-isopropylpipéridin-4-yl)amide d'acide 1-[5-(5-chlorothiophén-2-yl)isoxazol-3-ylméthyl]-5-pyridin-2-yl-1H-[1,2,4]triazole-3-carboxylique,
le (1-isopropylpipéridin-4-yl)amide d'acide 2-[5-(5-chlorothiophén-2-yl)isoxazol-3-ylméthyl]-5-pyridin-2-yl-2H-[1,2,4]triazole-3-carboxylique,
le(1-isopropylpipéridin-4-yl)amide d'acide 1-[5-(5-chlorothiophén-2-yl)isoxazol-3-ylméthyl]-5-phényl-1H-[1,2,4] triazole-3-carboxylique ou
le (1-isopropylpipéridin-4-yl)amide d'acide 2-[5-(5-chlorothiophén-2-yl)isoxazol-3-ylméthyl]-2H-tétrazole-5-carboxylique.

2. Préparation pharmaceutique comprenant un composé selon la revendication 1 sous toutes ses formes stéréoisomères et les mélanges de celles-ci dans un rapport quelconque et/ou ses sels physiologiquement tolérables et un support pharmaceutiquement acceptable.

3. Utilisation d'un composé selon la revendication 1, sous toutes ses formes stéréoisomères et des mélanges de celles-ci dans un rapport quelconque et/ou leurs sels physiologiquement tolérables, pour la production de produits pharmaceutiques destinés à influencer la coagulation du sang ou la fibrinolyse.

4. Utilisation selon la revendication 3 pour la formation de thrombus anormale, l'infarctus du myocarde aigu, les troubles cardiovasculaires, l'angine instable, le thromboembolisme, la fermeture d'un vaisseau aigu associée à une thérapie thrombolytique ou à l'angioplastie coronarienne transluminale percutanée (ACTP), les attaques ischémiques transitoires, l'accident cérébrovasculaire, la claudication intermittente, la greffe par pontage des artères coronaires ou périphériques, le rétrécissement de la lumière des vaisseaux, la resténose post-angioplastie coronaire ou veineuse, le maintien de l'ouverture de l'accès des vaisseaux chez les hémodialysés chroniques, la formation de thrombus pathologique se produisant dans les veines des extrémités inférieures suite à une intervention chirurgicale de l'abdomen, du genou ou de la hanche, un risque de thromboembolisme pulmonaire, ou la coagulopathie intravasculaire systémique disséminée se produisant dans les systèmes vasculaires au cours d'un choc septique, les infections virales ou le cancer, ou la réduction d'une réponse inflammatoire, la fibrinolyse, ou le traitement de maladies cardiaques coronariennes, l'infarctus du myocarde, l'angine de poitrine, la resténose vasculaire, par exemple, la resténose faisant suite à une angioplastie telle qu'une ACTP, le syndrome de détresse respiratoire de l'adulte, l'insuffisance d'organes multiples et le trouble de coagulation intravasculaire disséminé, la thrombose veineuse profonde ou veineuse proximale pouvant se produire suite à une opération chirurgicale.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- EP 987274 A
- WO 9529189 A
- WO 0200651 A
- WO 9206711 A
- WO 9612800 A
- WO 9747651 A
- WO 0107436 A2


### Non-patent literature cited in the description


- **A. E. P. ADANG ; J. B. M. REWINKEL.** *Drugs of the Future,* 2000, vol. 25, 369-383
- **CHEN ; PRUSOFF.** Biochem. Pharmacol. 1973, vol. 22, 3099-3108
- **I. H. SEGAL.** *Enzyme Kinetics,* 1975, 100-125
- **J. A. OSTREM et al.** *Biochemistry,* 1998, vol. 37, 1053-1059